# FASCICULE DE BREVET EUROPEEN

(11) **EP 0 678 014 B1**
(45) Date de publication et mention de la délivrance du brevet: **09.04.1997**
(21) Numéro de dépôt: 94904215.4
(22) Date de dépôt: 10.01.1994
(51) Int. Cl.: A61K 7/13, D06P 1/673, D06P 1/651

(54) **UTILISATION DE SEL DE MAGNESIUM DANS UN PROCEDE DE TEINTURE DES FIBRES KERATINIQUES METTANT EN OEUVRE LE 5,6-DIHYDROXYINDOLE OU L'UN DE SES DERIVES, PROCEDES ET COMPOSITIONS LES METTANT EN OEUVRE**
VERFAHREN VON MAGNESIUM SALZEN IN EINEM ZUM FÄRBEN VON KERATINFASERN IN KOMBINATION MIT 5,6-DIHYDROXYINDOLS
USE OF MAGNESIUM SALT IN A KERATIN FIBRE DYEING METHOD USING 5,6-DIHYDROXYINDOLE OR A DERIVATIVE THEREOF, AND METHODS AND COMPOSITIONS THEREFOR

(30) Priorité: 11.01.1993 FR 9300172
(43) Date de publication de la demande: 25.10.1995
(73) Titulaire: L'OREAL, 75008 Paris (FR)
(72) Inventeur: MAUBRU, Mireille, F-78400 Chatou (FR); AUDOUSSET, Marie-Pascale, F-92300 Levallois-Perret (FR); GIACOMONI, Paolo, F-95880 Enghien-les-Bains (FR); MARROT, Laurent, F-93190 Livry-Gargan (FR)
(74) Mandataire: Casalonga, Axel
(86) Numéro de dépôt international: FR9400026
(87) Numéro de publication internationale: WO9415576

(56) Documents cités:
- EP-A- 0 350 385
- EP-A- 0 415 802
- EP-A- 0 446 132
- EP-A- 0 462 883
- GB-A- 2 132 642

## Description

La présente invention est relative à l'utilisation d'un sel de magnésium dans un procédé de teinture des fibres kératiniques mettant en oeuvre le 5,6-dihydroxyindole ou l'un de ses dérivés, au procédé de teinture des fibres kératiniques et plus particulièrement des cheveux humains, consistant à utiliser un sel de magnésium different d'un nitrite ou d'un periodate et un colorant de la famille des 5,6-dihydroxyindole et aux- compositions tinctoriales mises en oeuvre.

La coloration des cheveux, de la peau et des poils, en particulier d'origine humaine, provient principalement des pigments mélaniques sécrétés par les mélanocytes. Ces pigments, d'origine naturelle, comprennent en particulier des eumélanines. Leur biosynthèse naturelle s'effectue en plusieurs étapes par polymérisation des produits d'oxydation d'un acide aminé : la tyrosine et l'un de ses produits d'oxydation est le 5,6-dihydroxyindole qui polymérise à son tour en eumélanines.

On a déjà proposé dans le passé de repigmenter les cheveux humains avec du 5,6-dihydroxyindole. Le 5,6-dihydroxyindole est généralement utilisé dans les compositions aqueuses avec lesquelles il est possible de teindre les fibres kératiniques et en particulier les cheveux, cette teinture pouvant être effectuée en plusieurs fois ou de façon progressive sans autre agent oxydant que l'oxygène de l'air. Il est possible de cette façon de repigmenter les cheveux en partant de nuances relativement claires par une application du produit, jusque dans des nuances de plus en plus soutenues par superposition des applications.

Le brevet EP-A-0 415 802 a pour objet un procédé de teinture de fibres kératiniques à base de monohydroxyindole et d'hydroxyindoles 5,6-disubstitués. Ce document prévoit l'utilisation de sels d'acide périodiques utilisés comme agent oxydant des dérivés indoliques.

On recherche, lors de la coloration des fibres kératiniques et plus particulièrement des cheveux, avec le 5,6-dihydroxyindole ou ses dérivés, l'obtention de nuances sombres et neutres qui n'évoluent pas au cours du temps, notamment lors de l'exposition à la lumière, à la transpiration ou à des lavages répétés.

La demande EP-A-0 350 585 a pour objet l'utilisation de nitrites de métaux alcalinoterreux à titre d'agent oxydant pour des dérivés indoliques.

Les "couleurs neutres" dans le système de notation L, a, b, se traduisent par de faibles valeurs des paramètres a et b.

La demanderesse a découvert, ce qui fait l'objet de l'invention, que l'utilisation d'un sel de magnésium dans un procédé de teinture des fibres kératiniques et en particulier des cheveux mettant en oeuvre du 5,6-dihydroxyindole ou ses dérivés, permettait d'obtenir une coloration présentant une ténacité améliorée vis-à-vis de la lumière. Ces teintures présentent également de bonnes résistances aux lavages répétés et à la transpiration.

L'invention a donc pour objet l'utilisation de sel de magnésium different d'un nitrite ou d'un periodate dans un procédé de coloration des fibres kératiniques et en particulier des cheveux, mettant en oeuvre le 5,6-dihydroxyindole ou ses dérivés.

Un autre objet de l'invention est constitué par le procédé de teinture mettant en oeuvre le 5,6-dihydroxyindole ou ses dérivés et un sel de magnésium different d'un nitrite ou d'un periodate.

L'invention a également pour objet une composition tinctoriale à base de 5,6-dihydroxyindole ou d'un de ses dérivés et d'un sel de magnésium different d'un nitrite ou d'un periodate.

D'autres objets de l'invention apparaîtront à la lecture de la description et des exemples qui suivent.

Selon l'invention, on utilise dans un procédé de coloration des fibres kératiniques et en particulier des cheveux humains, à l'aide d'une composition contenant dans un milieu approprié pour la teinture, du 5,6-dihydroxyindole et/ou un de ses dérivés répondant à la formule : 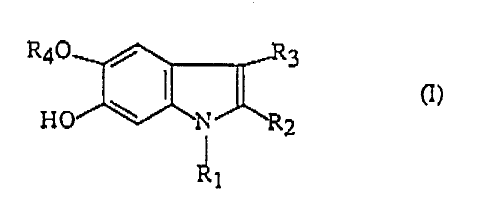 dans laquelle R₁, R₂ R₃, R₄, identiques ou différents, désignent hydrogène ou alkyle inférieur on C₁-C₄, R₁, R₂, R₃ désignant hydrogène lorsque R₄ désigne alkyle inférieur, un sel minéral ou organique simple ou polymérique du magnésium different d'un nitrite ou d'un periodate ou leurs mélanges soluble(s) dans le milieu utilisé pour son application sur les fibres kératiniques.

Dans la formule (I), le radical alkyle inférieur désigne de préférence méthyle ou éthyle et R₁, R₂ et R₃ ne désignent pas simultanément alkyle inférieur.

Les composés de formule (I) préférés sont choisis parmi le 5,6-dihydroxyindole, le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole, Un composé particulièrement préféré est le 5,6-dihydroxyindole.

Les composés de formule (I) sont généralement mis en oeuvre dans des proportions suffisantes pour repigmenter les fibres et en particulier les cheveux. Ces proportions sont comprises entre 0,01 et 5% et de préférence entre 0,2 et 3% en poids par rapport au poids total de la composition.

Les sels de magnésium utilisables conformément à l'invention ne sont ni des nitrites, ni des périodates.

Ce sont plus particulièrement des sels de magnésium d'un acide choisi parmi :
- les hydracides halogénés,
- les oxyacides dérivés du soufre,
- les acides mono-, di- ou tricarboxyliques contenant moins de 8 atomes de carbone et éventuellement hydroxylés,
- les acides sulfoniques organiques contenant moins de 8 atomes de carbone.

Les sels de magnésium préférés sont plus particulièrement le chlorure, l'acétate et le sulfate de magnésium.

Le sel de magnésium peut être utilisé et être appliqué sur les fibres kératiniques et en particulier les cheveux humains, soit avant l'application de la composition contenant le 5,6-dihydroxyindole et/ou l'un de ses dérivés, soit simultanément ou dans la composition contenant le 5,6-dihydroxyindole ou l'un de ses dérivés ou postérieurement à l'application de la composition contenant le 5,6-dihydroxyindole ou l'un de ses dérivés.

Les compositions contenant le 5,6-dihydroxyindole et/ou ses dérivés de formule (I) et/ou le sel de magnésium, different d'un nitrite ou d'un periodate sont des compositions aqueuses ou des compositions dont le milieu est un mélange d'eau et d'un solvant, cosmétiquement acceptable lorsque la composition est appliquée sur les cheveux humains.

Le sel de magnésium est généralement mis en oeuvre dans des proportions comprises entre 0,05 et 20% en poids par rapport au poids total de la composition et de préférence entre 0,2 et 10% en poids par rapport au poids total de la composition.

Les compositions contenant le sel de magnésium et/ou le 5,6-dihydroxyindole et/ou l'un de ses dérivés, peuvent avoir un pH de 3' à 10 et de' préférence de 6 à 9. Le pH est ajusté par des agents acidifiants et/ou alcalinisants connus en eux-mêmes.

Lorsque les compositions contenant le 5,6-dihydroxyindole et/ou ses dérivés de formule (I) ainsi que les compositions contenant le sel de magnésium, contiennent un mélange d'eau et de solvant, celui-ci est choisi parmi les alcools inférieurs en C₁-C₄ tels que l'alcool éthylique, l'alcool propylique, l'alcool isopropylique, l'alcool tertiobutylique; les glycols tels que l'éthylèneglycol, le propylèneglycol; les éthers de glycols tels que les éthers monométhylique, monoéthylique ou monobutylique de l'éthylèneglycol, les monométhyléthers du propylèneglycol et du dipropylèneglycol, les esters inférieurs comme le lactate de méthyle et l'acétate de monoéthyléther de l'éthylèneglycol.

Les solvants particulièrement préférés sont choisis parmi l'alcool éthylique et le propylèneglycol.

Les solvants sont généralement présents dans des proportions comprises entré 0,5 et 50% en poids et de préférence 2 à 20% en poids par rapport au poids total de la composition.

Les compositions utilisées selon l'invention peuvent se présenter sous la forme de lotions épaissies ou non, de gels, de shampooings, d'émulsions telles que les laits et les crèmes ou elles peuvent être propulsées sous forme de mousses aérosols.

Ces compositions contenant le 5,6-dihydroxyindole et/ou ses dérivés de formule (I) peuvent contenir des agents alcalinisants ou acidifiants cosmétiquement acceptables, permettant d'ajuster la valeur du pH entre 3 et 10 et de préférence entre 5 et 7.

Une forme de réalisation particulièrement préférée consiste à utiliser un agent régulateur de pH à deux composants tels que par exemple les couples suivants : phosphate acide dipotassique/phosphate diacide de potassium ou bien triéthanolamine/acide tartrique.

Les compositions contenant le 5,6-dihydroxyindole et/ou ses dérivés de formule (I) et/ou le sel de magnésium different d'un nitrite ou d'un periodate, peuvent être épaissies avec des agents épaississants utilisés de préférence dans des proportions en poids comprises entre 0,1 et 5% et en particulier entre 0,5 et 3% par rapport au poids total de la composition.

Les épaississants sont plus particulièrement choisis parmi l'alginate de sodium, la gomme arabique, la gomme de guar, les hétérobiopolysaccharides comme la gomme de xanthane, les scléroglucanes, les dérivés de cellulose tels que la méthylcellulose, l'hydroxyéthylcellulose, l'hydroxypropylméthylcellulose, les sels de sodium de la carboxyméthylcellulose, les polymères d'acide acrylique, les cires telles que par exemple un mélange d'alcool cétylstéarylique C₁₆/C₁₈/alcool cétylstéarylique C₁₆/C₁₈ polyoxyéthyléné, ou encore des agents épaississants minéraux tels que la bentonite.

Les compositions conformes à l'invention peuvent également renfermer des agents tensio-actifs anioniques, cationiques, non-ioniques, amphotères ou leurs mélanges.

Parmi ces agents tensio-actifs, on peut plus particulièrement citer les alkylbenzènesulfonates, les alkylnaphtalènesulfonates, les sulfates, les éthersulfates et les sulfonates d'alcools gras, les sels d'ammonium quaternaires tels que le bromure de triméthylcétylammonium, le bromure de cétylpyridinium, les éthanolamides d'acides gras éventuellement oxyéthylénés, les acides, les alcools ou les amines polyoxyéthylénés, les alcools polyglycérolés, les alkylphénols polyoxyéthylénés ou polyglycérolés, les alkylsulfates polyoxyéthylénés, les alkylpolyglycosides.

Les compositions contenant le sel de magnésium et/ou le 5,6-dihydroxyindole et/ou ses dérivés, peuvent également contenir des polymères anioniques, cationiques, non ioniques et/ou amphotères.

Une forme de réalisation consiste à utiliser la composition contenant le 5,6-dihydroxyindole et/ou ses dérivés de formule (I), en présence éventuellement du sel de magnésium sous forme de mousse. Dans cette forme de réalisation, la composition tinctoriale peut être stockée dans un dispositif aérosol en présence d'un agent propulseur classique.

Ces compositions contiennent des générateurs de mousse tels qu'un agent tensio-actif ou un polymère moussant ou un mélange de ceux-ci.

Les compositions conformes à l'invention peuvent également renfermer tout autre additif habituellement utilisé dans des compositions tinctotiales pour fibres kératiniques cosmétiquement acceptables lorsqu'ils sont appliqués sur les cheveux, tel que des agents de pénétration, des agents gonflants, des agents séquestrants, des agents filmogènes, des agents anti-oxydants, des électrolytes, des parfums, des agents nacrants.

Lorsque la composition est utilisée sous forme de mousse, il est évident que ces adjuvants ne doivent pas empêcher la formation de mousse après distribution du produit sous pression à partir du dispositif aérosol.

Le procédé conforme à l'invention consiste à appliquer sur les fibres kératiniques et en particulier les cheveux humains, au moins un sel de magnésium different d'un nitrite ou d'un periodate et au moins du 5,6-dihydroxyindole et/ou un de ses dérivés répondant à la formule (I) sur les fibres, le sel de magnésium étant appliqué soit préalablement à l'application du 5,6-dihydroxyindole et/ou de ses dérivés de formule (I) sur les fibres, soit simultanément, soit postérieurement à l'application de la composition contenant le 5,6-dihydroxyindole et/ou l'un de ses dérivés de formule (I), le sel de magnésium et le 5,6-dihydroxyindole et/ou ses dérivés de formule (I) étant appliqués à l'aide de compositions les contenant dans un milieu approprié pour la teinture tels que définis ci-dessus.

Les compositions mises en oeuvre au cours du procédé sont telles que définies ci-dessus.

Un autre objet de l'invention est constitué par une composition tinctoriale pour fibres kératiniques, en particulier pour cheveux humains, caractérisée par le fait qu'elle contient dans un milieu approprié pour la teinture, au moins un sel de magnésium tel que défini ci-dessus et au moins le 5,6-dihydroxyindole et/ou un de ses dérivés répondant à la formule (I) définie ci-dessus.

Cette composition contient de préférence le sel de magnésium dans des proportions comprises entre 0,05 et 20% en poids par rapport au poids total de la composition et de préférence entre 0,2 et 10% en poids et le 5,6-dihydroxyindole et/ou ses dérivés de formule (I) dans des proportions comprises entre 0,01 et 5% en poids et de préférence entre 0,2 et 3% en poids par rapport au poids total de la composition.

Le procédé de teinture conforme à l'invention est mis en oeuvre de préférence sans utilisation d'un autre agent oxydant que l'oxygène de l'air.

Les exemples suivants sont destinés à illustrer l'invention sans pour autant présenter un caractère limitatif.

### EXEMPLE 1

| **PRE-TRAITEMENT** | |
|---|---|
| - MgCl₂, 6H₂O | 4,3 g |
| - Triéthanolamine qs pH = 7 | |
| - Eau déminéralisée | qsp 100 g |

| **GEL COLORANT** | |
|---|---|
| - 5,6-dihydroxyindole | 0,5 g |
| - Alcool éthylique à 96° | 10 g |
| - Hydroxyéthylcellulose vendue sous la dénomination "KLUCEL G" par la Société AQUALON | 2 g |
| - Alkyl(C₈-C₁₀)polyglycoside en solution aqueuse a 60% de MA, vendu sous la dénomination "TRITON CG 110" par la Société ROHM & HAAS | 3,5 g |
| - Nonylphénol oxyéthyléné (9 moles d'oxyde d'éthylène), vendu sous la dénomination "RHODIASURF NP 90 R" par la Société RHONE POULENC | 0,1 g |
| - Acide tartrique | 0,3 g |
| - Triéthanolamine qs pH = 8,5 | |
| - Eau déminéralisée | qsp 100 g |

La lotion de prétraitement est appliquée sur des cheveux naturels gris à 90% de blancs, à raison de 1 g pour 1,5 g de cheveux.

Après imprégnation des cheveux et sans rinçage intermédiaire, 5 g de gel colorant sont appliqués sur les cheveux.

Après 10 minutes de pose, les cheveux sont rincés et séchés.

On renouvelle l'application une fois.

Les cheveux sont teints uniformément en gris et présentent une bonne résistance à la lumière, la couleur restant dans les tons neutres.

### EXEMPLE 2

| **GEL COLORANT** | |
|---|---|
| - 5,6-dihydroxyindole | 0,5 g |
| - Alcool éthylique à 96° | 10 g |
| - Hydroxyéthylcellulose vendue sous la dénomination "KLUCEL G" par la Société AQUALON | 2 g |
| - Alkyl(C₈-C₁₀)polyglycoside en solution aqueuse à 60% de MA, vendu sous la dénomination "TRITON CG 110" par la Société ROHM & HAAS | 3,5 g |
| - Nonylphénol oxyéthyléné (9 moles d'oxyde d'éthylène), vendu sous la dénomination "RHODIASURF NP 90 R" par la Société RHONE POULENC | 0,1 g |
| - Acide tartrique | 0,3 g |
| - Triéthanolamine qs pH = 8,5 | |
| - Eau déminéralisée | qsp 100 g |

| **POST-TRAITEMENT** | |
|---|---|
| - MgCl₂, 6H₂O | 4,3 g |
| - Ethanol à 96° | 17 g |
| - Poly-β-alanine en solution aqueuse à 20% de MA | 2,5 g |
| - Triéthanolamine qs pH = 7 | |
| - Eau déminéralisée | qsp 100 g |

La composition colorante est appliquée sur des cheveux naturels gris à 90% de blancs, à raison de 5 g pour 1,5 g de cheveux.

Après 10 minutes de pose, les cheveux sont rincés et essorés.

1 g de lotion de post-traitement est alors réparti sur la mèche. Les cheveux sont séchés après 5 minutes de pose et sans rinçage terminal.

Après deux applications, les cheveux sont teints uniformément en gris et présentent une bonne résistance à la lumière.

### EXEMPLE 3

| **GEL COLORANT** | |
|---|---|
| - 5,6-dihydroxyindole | 0,5 g |
| - MgCl₂,6H₂O | 0,86 g |
| - Alcool éthylique à 96° | 10 g |
| - Hydroxyéthylcellulose vendue sous la dénomination "KLUCEL G" par la Société AQUALON | 2 g |
| - Alkyl(C₈-C₁₀)polyglycoside en solution aqueuse à 60% de MA, vendu sous la dénomination "TRITON CG 110" par la Société ROHM & HAAS | 3,5 g |
| - Nonylphénol oxyéthyléné (9 modes d'oxyde d'éthylène), vendu sous la dénomination "RHODIASURF NP 90 R" par la Société RHONE POULENC | 0,1 g |
| - Acide tartrique | 0,3 g |
| - Triéthanolamine qs pH = 8,5 | |
| - Eau déminéralisée | qsp 100 g |

La composition colorante est appliquée sur des cheveux gris à 90% de blancs naturels, à raison de 10 g pour 3 g de cheveux.

Après 10 minutes de pose, les cheveux sont rincés et séchés.

Après deux applications, les cheveux sont teints uniformément en gris et présentent une bonne résistance à la lumière.

### EXEMPLE 4

| **PRE-TRAITEMENT** | |
|---|---|
| - MgSO₄,7H₂O | 2,4 g |
| - Ethanol | 10 g |
| - Triéthanolamine qs pH = 7 | |
| - Eau déminéralisée | qsp 100 g |

| **GEL COLORANT** | |
|---|---|
| - 5,6-dihydroxyindole | 0,5 g |
| - Alcool éthylique à 96° | 10 g |
| - Hydroxyéthylcellulose vendue sous la dénomination "KLUCEL G" par la Société AQUALON | 2 g |
| - Alkyl(C₈-C₁₀)polyglycoside en solution aqueuse à 60% de MA, vendu sous la dénomination "TRITON CG 110" par la Société ROHM & HAAS | 3,5 g |
| - Nonylphénol oxyéthyléné (9 moles d'oxyde d'éthylène), vendu sous la dénomination "RHODIASURF NP 90 R" par la Société RHONE POULENC | 0,1 g |
| - Acide tartrique | 0,3 g |
| - Triéthanolamine qs pH = 8,5 | |
| - Eau déminéralisée | qsp 100 g |

La lotion de pré-traitement est appliquée sur des cheveux gris naturels à 90% de blancs à raison de 2 g pour 3 g de cheveux.

Après imprégnation des cheveux et sans rinçage intermédiaire, 10 g de gel colorant sont appliqués sur les cheveux.

Après 10 minutes de pose, les cheveux sont rincés et séchés.

Après deux applications, les cheveux sont teints uniformément en gris et présentent une bonne résistance à la lumière.

### EXEMPLE 5

| **PRE-TRAITEMENT** | |
|---|---|
| - Mg(CH₃COO)₂,4H₂O | 4,3 g |
| - Ethanol | 10 g |
| - Acide lactique qs pH = 7 | |
| - Eau déminéralisée | qsp 100 g |

| **GEL COLORANT** | |
|---|---|
| - 5,6-dihydroxyindole | 0,5 g |
| - Alcool éthylique à 96° | 10 g |
| - Hydroxyéthylcellulose vendue sous la dénomination "KLUCEL G" par la Société AQUALON | 2 g |
| - Alkyl(C₈-C₁₀)polyglycoside en solution aqueuse à 60% de MA, vendu sous la dénomination "TRITON CG 110" par la Société ROHM & HAAS | 3,5 g |
| - Nonylphénol oxyéthyléné (9 moles d'oxyde d'éthylène), vendu sous la dénomination "RHODIASURF NP 90 R" par la Société RHONE POULENC | 0,1 g |
| - Acide tartrique | 0,3 g |
| - Triéthanolamine qs pH = 8,5 | |
| - Eau déminéralisée | qsp 100 g |

La lotion de pré-traitement est appliquée sur des cheveux gris naturels à 90% de blancs à raison de 2 g pour 3 g de cheveux.

Après imprégnation des cheveux et sans rinçage intermédiaire, 10 g de gel colorant sont appliqués sur les cheveux.

Après 10 minutes de pose, les cheveux sont rincés et séchés.

Après deux applications, les cheveux sont teints uniformément en gris et présentent une bonne résistance à la lumière.

## Revendications

1. Utilisation d'un sel minéral ou organique simple ou polymérique de magnésium, différent d'un nitrite ou d'un périodate, soluble dans le milieu utilisé pour son application, dans un procédé de coloration des fibres kératiniques et en particulier des cheveux humains, mettant en oeuvre à titre d'agent colorant le 5,6-dihydroxyindole et/ou ses dérivés répondant à la formule (I) : 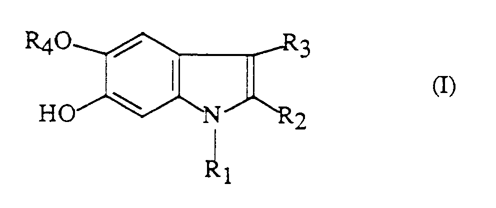 dans laquelle R₁, R₂ R₃, R₄, identiques ou différents, désignent hydrogène ou alkyle inférieur en C₁-C₄, R₁, R₂, R₃ désignant hydrogène lorsque R₄ désigne alkyle en C₁-C₄.

2. Utilisation selon la revendication 1, caractérisée par le fait que le sel de magnésium est un sel d'un acide choisi parmi les hydracides halogénés, les oxyacides dérivés du soufre, les acides mono-, di- ou tricarboxyliques contenant moins de 8 atomes de carbone et éventuellement hydroxylés, les acides sulfoniques organiques contenant moins de 8 atomes de carbone.

3. Utilisation selon la revendication 1 ou 2, caractérisée par le fait que le sel de magnésium est un chlorure, un acétate ou un sulfate de magnésium.

4. Utilisation selon l'une quelconque des revendications 1 à 3, caractérisée par le fait que le 5,6-dihydroxyindole de formule (I) est choisi parmi le 5,6-dihydroxyindole, le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole.

5. Procédé de teinture des fibres kératiniques et en particulier des cheveux humains, caractérisé par le fait que l'on applique sur ces fibres (A) au moins un sel minéral ou organique, simple ou polymérique de magnésium, différent d'un nitrite ou d'un périodate, soluble dans le milieu utilisé pour son application; (B) au moins le 5,6-dihydroxyindole et/ou l'un de ses dérivés, répondant à la formule (I) : 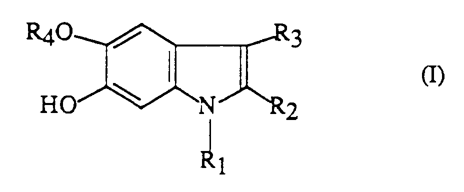 dans laquelle R₁, R₂ R₃, R₄, identiques ou différents, désignent hydrogène ou alkyle inférieur en C₁-C₄, R₁, R₂, R₃ désignant hydrogène lorsque R₄ désigne alkyle en C₁-C₄, le sel de magnésium étant appliqué soit préalablement à l'application du 5,6-dihydroxyindole et/ou de ses dérivés de formule (I), soit simultanément, soit postérieurement, le sel de magnésium et le 5,6-dihydroxyindole et/ou ses dérivés de formule (I) étant appliqués à l'aide de compositions les contenant dans un milieu approprié pour la coloration des fibres kératiniques et en particulier des cheveux humains.

6. Procédé selon la revendication 5, caractérisé par le fait que le sel de magnésium est un sel d'un acide choisi parmi les hydracides halogénés, les oxyacides dérivés du soufre, les acides mono-, di- ou tricarboxyliques contenant moins de 8 atomes de carbone et éventuellement hydroxylés, les acides sulfoniques organiques contenant moins de 8 atomes de carbone.

7. Procédé selon la revendication 5 ou 6, caractérisé par le fait que le sel de magnésium et le 5,6-dihydroxyindole et/ou ses dérivés de formule (I), sont appliqués simultanément sur les fibres kératiniques et en particulier les cheveux humains, à l'aide d'une même composition les contenant dans un milieu approprié pour la teinture.

8. Procédé selon l'une quelconque des revendications 5 à 7, caractérisé par le fait que le 5,6-dihydroxyindole et/ou ses dérivés de formule (I) sont présents dans la composition les contenant, dans des proportions comprises entre 0,01 et 5% en poids et de préférence entre 0,2 et 3% en poids par rapport au poids total de la composition.

9. Procédé selon l'une quelconque des revendications 5 à 8, caractérisé par le fait que la composition contenant le 5,6-dihydroxyindole et/ou ses dérivés de formule (I) a un pH compris entre 3 et 10, ajusté à l'aide d'un agent alcalinisant et/ou acidifiant.

10. Procédé selon la revendication 9, caractérisé par le fait que le pH est ajusté à l'aide d'un agent régulateur de pH à deux composants.

11. Procédé selon la revendication 10, caractérisé par le fait que l'agent régulateur de pH à deux composants est constitué par les couples acide tartrique/triéthanolamine ou bien phosphate acide dipotassique/ phosphate diacide de potassium.

12. Procédé selon l'une quelconque des revendications 5 à 11, caractérisé par le fait que le sel de magnésium est présent dans les compositions le contenant dans des proportions comprises entre 0,05 et 20% en poids et de préférence entre 0,2 et 10% en poids par rapport au poids total de la composition.

13. Procédé selon la revendication 12, caractérisé par le fait que le pH de la composition contenant le sel de magnésium est compris entre 3 et 10 et de préférence entre 6 et 9.

14. Procédé selon l'une quelconque des revendications 5 à 11, caractérisé par le fait que la composition contenant le 5,6-dihydroxyindole et/ou ses dérivés de formule (I), est appliquée sous forme d'une lotion épaissie ou non, d'un gel, d'une émulsion ou d'une mousse dispensée à partir d'un dispositif aérosol contenant la composition en présence d'un agent propulseur.

15. Procédé selon l'une quelconque des revendications 5 à 14, caractérisé par le fait que les compositions contenant le sel de magnésium et/ou le 5,6-dihydroxyindole et/ou ses dérivés de formule (I), contiennent un adjuvant cosmétiquement acceptable choisi parmi les agents épaississants et/ou les polymères et/ou les agents tensioactifs, les agents de pénétration, les agents gonflants, les agents séquestrants, les agents filmogènes, les agents antioxydants, les électrolytes, les parfums, les agents nacrants.

16. Composition tinctoriale pour fibres kératiniques et en particulier pour cheveux humains, caractérisée par le fait qu'elle contient au moins un sel minéral ou organique simple ou polymérique de magnésium, différent d'un nitrite ou d'un périodate, soluble dans le milieu approprié pour la teinture, et au moins un dérivé de 5,6-dihydroxyindole répondant à la formule (I) : 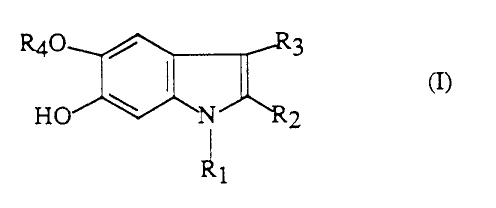 dans laquelle R₁, R₂ R₃, R₄, identiques ou différents, désignent hydrogène ou alkyle inférieur en C₁-C₄, R₁, R₂, R₃ désignant hydrogène lorsque R₄ désigne alkyle inférieur dans un milieu approprié pour la teinture des fibres kératiniques et en particulier pour les cheveux humains.

17. Composition selon la revendication 16, caractérisée par le fait que le sel de magnésium est un sel d'un acide choisi parmi les hydracides halogénés, les oxyacides dérivés du soufre, les acides mono-, di- ou tricarboxyliques contenant moins de 8 atomes de carbone et éventuellement hydroxylés, les acides sulfoniques organiques contenant moins de 8 atomes de carbone.

## Claims

1. Use of a simple or polymeric organic or inorganic magnesium salt, other than a nitrite or a periodate, which is soluble in the medium used for its application, in a process for colouring keratinous fibres and in particular human hair, employing, as colouring agent, 5,6-dihydroxyindole and/or its derivatives corresponding to the formula (I): 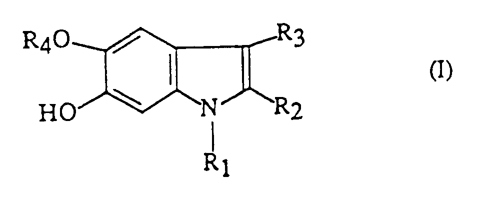 in which R₁, R₂, R₃ and R₄, which are identical or different, denote hydrogen or lower C₁-C₄ alkyl, R₁, R₂ and R₃ denoting hydrogen when R₄ denotes C₁-C₄ alkyl.

2. Use according to Claim 1, characterized in that the magnesium salt is a salt of an acid chosen from halogenated hydracids, oxyacids derived from sulphur, mono-, di- or tricarboxylic acids containing less than 8 carbon atoms and which are optionally hydroxylated, or organic sulphonic acids containing less than 8 carbon atoms.

3. Use according to Claim 1 or 2, characterized in that the magnesium salt is a magnesium chloride, a magnesium acetate or a magnesium sulphate.

4. Use according to any one of Claims 1 to 3, characterized in that the 5,6-dihydroxyindole of formula (I) is chosen from 5,6-dihydroxyindole, 2-methyl-5,6-dihydroxyindole, 3-methyl-5,6-dihydroxyindole, 1-methyl-5,6-dihydroxyindole, 2,3-dimethyl-5,6-dihydroxyindole or 5-methoxy-6-hydroxyindole.

5. Process for dyeing keratinous fibres and in particular human hair, characterized in that there is applied to these fibres (A) at least one simple or polymeric organic or inorganic magnesium salt, other than a nitrite or a periodate, which is soluble in the medium used for its application; (B) at least 5,6-dihydroxyindole and/or one of its derivatives, corresponding to the formula (I): 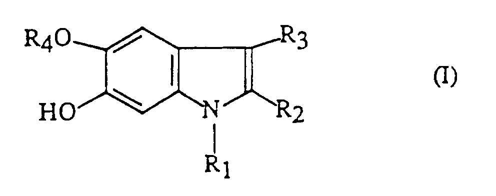 in which R₁, R₂, R₃ and R₄, which are identical or different, denote hydrogen or lower C₁-C₄ alkyl, R₁, R₂ and R₃ denoting hydrogen when R₄ denotes C₁-C₄ alkyl, the magnesium salt being applied either prior to application of 5,6-dihydroxyindole and/or of its derivatives of formula (I), or simultaneously, or subsequently, the magnesium salt and 5,6-dihydroxyindole and/or its derivatives of formula (I) being applied using compositions containing them in a medium appropriate for colouring keratinous fibres and in particular human hair.

6. Process according to Claim 5, characterized in that the magnesium salt is a salt of an acid chosen from halogenated hydracids, oxyacids derived from sulphur, mono-, di- or tricarboxylic acids containing less than 8 carbon atoms and which are optionally hydroxylated, or organic sulphonic acids containing less than 8 carbon atoms.

7. Process according to Claim 5 or 6, characterized in that the magnesium salt and 5,6-dihydroxyindole and/or its derivatives of formula (I) are applied simultaneously to keratinous fibres and in particular human hair, using the same composition containing them in a medium appropriate for dyeing.

8. Process according to any one of Claims 5 to 7, characterized in that 5,6-dihydroxyindole and/or its derivatives of formula (I) are present in the composition containing them in proportions of between 0.01 and 5 % by weight and preferably between 0.2 and 3 % by weight with respect to the total weight of the composition.

9. Process according to any one of Claims 5 to 8, characterized in that the composition containing 5,6-dihydroxyindole and/or its derivatives of formula (I) has a pH of between 3 and 10, adjusted using a basifying and/or acidifying agent.

10. Process according to Claim 9, characterized in that the pH is adjusted using a two-component pH-regulating agent.

11. Process according to Claim 10, characterized in that the two-component pH-regulating agent comprises tartaric acid/triethanolamine or else dipotassium hydrogenphophate/potassium dihydrogenphosphate pairs.

12. Process according to any one of Claims 5 to 11, characterized in that the magnesium salt is present in the compositions containing it in proportions of between 0.05 and 20 % by weight and preferably between 0.2 and 10 % by weight with respect to the total weight of the composition.

13. Process according to Claim 12, characterized in that the pH of the composition containing the magnesium salt is between 3 and 10 and preferably between 6 and 9.

14. Process according to any one of Claims 5 to 11, characterized in that the composition containing 5,6-dihydroxyindole and/or its derivatives of formula (I) is applied in the form of a thickened or nonthickened lotion, of a gel, of an emulsion or of a foam dispensed from an aerosol device containing the composition in the presence of a propellent.

15. Process according to any one of Claims 5 to 14, characterized in that the compositions containing the magnesium salt and/or 5,6-dihydroxyindole and/or its derivatives of formula (I) contain a cosmetically acceptable adjuvant chosen from thickening agents and/or polymers and/or surface-active agents, penetrating agents, swelling agents, sequestering agents, film-forming agents, antioxidizing agents, electrolytes, fragrances or pearlescent agents.

16. Dyeing composition for keratinous fibres and in particular for human hair, characterized in that it contains at least one simple or polymeric organic or inorganic magnesium salt, other than a nitrite or a periodate, which is soluble in the medium appropriate for dyeing, and at least one 5,6-dihydroxyindole derivative corresponding to the formula (I): 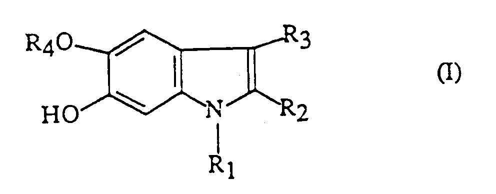 in which R₁, R₂, R₃ and R₄, which are identical or different, denote hydrogen or lower C₁-C₄ alkyl, R₁, R₂ and R₃ denoting hydrogen when R₄ denotes lower alkyl, in a medium appropriate for dyeing keratinous fibres and in particular for human hair.

17. Composition according to Claim 16, characterized in that the magnesium salt is a salt of an acid chosen from halogenated hydracids, oxyacids derived from sulphur, mono-, di- or tricarboxylic acids containing less than 8 carbon atoms and which are optionally hydroxylated, or organic sulphonic acids containing less than 8 carbon atoms.

## Patentansprüche

1. Verwendung eines Mineralsalzes oder eines einfachen oder mehrbasgen organischen Salzes von Magnesium, welche sich von einem Nitrit oder Perjodat unterscheiden und in dem zu deren Anwendung eingesetzten Milieu löslich sind, in einem Verfahren zur Färbung keratinischer Fasern und insbesondere der menschlichen Haare, wobei man als Färbemittel 5,6-Dihydroxyindol und/oder seine Derivate der Formel (I) einsetzt: 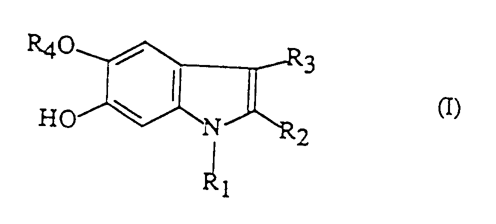 worin R₁, R₂, R₃ und R₄ gleich oder verschieden, Wasserstoff oder einen C₁₋₄-Niedrigalkylrest bedeuten, wobei R₁, R₂ und R₃ Wasserstoff bedeuten, wenn R₄ einen C₁₋₄-Niedrgalkylrest bedeutet.

2. Verwendung gemäß Anspruch 1,
dadurch **gekennzeichnet**, daß
das Magnesiumsalz ein Salz einer Säure ist, die aus halogenierten Wasserstoffsäuren, von Schwefel abgeleiteten Oxisäuren, Mono-, Di- oder Tricarboxylsäuren, die mindestens 8 Kohlenstoffatome enthalten und gegebenenfalls hydroxyliert sind, und aus organischen Sulfonsäuren mit mindestens 8 Kohlenstoffatomsäuren ausgewählt ist.

3. Verwendung gemäß Anspruch 1 oder 2,
dadurch **gekennzeichnet**, daß
das Magnesiumslaz ein Chlorid, Acetat oder Sulfat von Magnesium ist.

4. Verwendung gemäß jedem der Ansprüche 1 bis 3,
dadurch **gekennzeichnet**, daß
die 5,6-Dihydroxyindole der Formel (I) aus 5,6-Dihydroxyindol, 2-Methyl-5,6-dihydroxyindol, 3-Methyl-5,6-Dihydroxyindol, 1-Methyl-5,6-dihydroxyindol, 2,3-Dimethyl-5,6-dihydroxyindol und aus 5-Methoxy-6-hydroxyindol ausgewählt sind.

5. Verfahren zur Färbung keratinischer Fasern und insbesondere der menschlichen Haare,
dadurch **gekennzeichnet**, daß
man auf diese Fasern (A) mindestens ein Mineralsalz oder ein einfaches oder mehrbasiges organisches Salz von Magnesium, welche sich von einem Nitrit oder Perjodat unterscheiden und in dem zu deren Anwendung eingesetzten Milieu löslich sind, und (B) mindestens 5,6-Dihydroxyindol und/oder eines seiner Derivate derFormel (I) aufbringt: 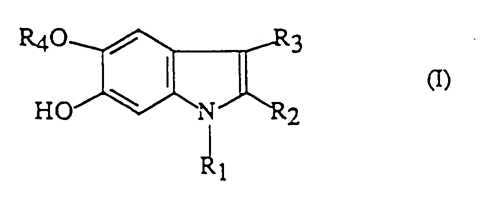 worin R₁, R₂, R₃ und R₄ gleich oder verschieden, Wasserstoff oder einen C₁₋₄-Niedrigalkylrest bedeuten, wobei R₁, R₂ und R₃ Wasserstoff bedeuten, wenn R₄ einen C₁₋₄-Niedrgalkylrest bedeutet, wobei das Magnesiumsalz entweder vor der Aufbringung des 5,6-Dihydroxyindols und/oder seiner Derivate der Formel (I) oder gleichzeitig oder danach aufgebracht wird, und wobei das Magnesiumsalz und das 5,6-Dihydroxyindol und/oder seine Derivate der Formel (I) mit Zusammensetzungen aufgebracht werden, die diese in einem zur Färbung der keratinischen Fasern und insbesondere der menschlichen Haare geeigneten Milieu enthalten.

6. Verfahren gemäß Anspruch 5,
dadurch **gekennzeichnet**, daß
das Magnesiunsalz ein Salz einer Säure ist, die aus halogenierten Wasserstoffsäuren, aus von Schwefel abgeleiteten Oxisäuren, aus Mono-, Di- oder Tricarboxylsäuren, die mindestens 8 Kohlenstoffatome enthalten und gegebenenfalls hydroxyliert sind, und aus organischen Sulfonsäuren mit mindestens 8 Kohlenstoffatomen ausgewählt ist.

7. Verfahren gemäß Anspruch 5 oder 6,
dadurch **gekennzeichnet**, daß
das Magnesiumsalz und das 5,6-Dihydroxyindol und/oder seine Derivate der Formel (I) gleichzeitig auf die keratinischen Fasern und insbesondere die menschlichen Haare mittels einer Zusammensetzung aufgebracht werden, die diese in einem zur Färbung geeigneten Milieu enthalten.

8. Verfahren gemäß einem der Ansprüche 5 bis 7,
dadurch **gekennzeichnet**, daß
das 5,6-Dihydroxyindol und/oder seine Derivate der Formel (I) in der diese enthaltenden Zusammensetzung in Mengenanteilen von 0,01 bis 5 und vorzugsweise von 0,2 bis 3 Gew.% vorhanden sind, bezogen auf das Gesamtgewicht der Zusammensetzung.

9. Verfahren gemäß eniem der Ansprüche 5 bis 8,
dadurch **gekennzeichnet**, daß
die das 5,6-Dihydroxyindol und/oder seine Derivate der Formel (I) enthaltende Zusammensetzung einen pH-Wert von 3 bis 10 aufweist, der mit einem alkalisch und/oder sauer stellenden Mittel eingestellt ist.

10. Verfahren gemäß Anspruch 9,
dadurch **gekennzeichnet**, daß
der pH-Wert mit einem Mittel zur Regulierung des pH-Wertes aus 2 Bestandteilen eingestellt wird.

11. Verfahren gemäß Anspruch 10,
dadurch **gekennzeichnet**, daß
das Mittel aus zwei Bestandteilen zur Regulierung des pH-Wertes aus den beiden Paaren aus Weinsäure/Triethanolamin oder auch aus Dikaliumhydrogenphosphat/Kaliumdihydrogenphosphat zusammgensetzt ist.

12. Verfahren gemäß einem der Ansprüche 5 bis 11,
dadurch **gekennzeichnet**, daß
das Magnesiumsalz in den dieses enthaltenden Zusammensetzungen in Mengenanteilen von 0,05 bis 20 und vorzugsweise von 0,2 bis 10 Gew.% vorhanden ist, bezogen auf das Gesamtgewicht der Zusammensetzung.

13. Verfahren gemäß Anspruch 12,
dadurch **gekennzeichnet**, daß
der pH-Wert der das Magnesiumsalz enthaltenden Zusammensetzung 3 bis 10 und vorzugsweise 6 bis 9 beträgt.

14. Verfahren gemäß einem der Anprüche 5 bis 11,
dadurch **gekennzeichnet**, daß
die das 5,6-Dihydroxyindol und/oder seine Derivate der Formel (I) enthaltende Zusammensetzung in Form einer gegebenenfalls verdickten Lotion, eines Gels, einer Emulsion oder eines Schaumes aufgebracht wird, der aus einer Aerosol-Vorrichtung aufgetragen wird, die die Zusammensetzung in Gegenwart eines Treibmittels enthält.

15. Verfahren gemäß einem der Ansprüche 5 bis 14,
dadurch **gekennzeichnet**, daß
die Zusammensetzungen, die das Magnesiumsalz und/oder das 5,6-Dihydroxyindol und/oder seine Derivate der Formel (I) enthalten, einen kosmetisch geeigneten Hilfsstoff enthalten, der aus Verdickungsmiteln und/oder Polymeren und/oder oberflächenaktiven Mitteln, aus Eindringmitteln, Lockerungsmitteln, Sequestriermitteln, filmbildenden Mitteln, Antioxidantien, Elektrolyten, Parfüm-Produkten und aus Perlmuttglanzmitteln ausgewählt ist.

16. Färbezusammensetzung für keratinische Fasern und insbesondere für menschliche Haare,
dadurch **gekennzeichnet**, daß
sie mindestens ein Mineralsalz oder ein einfaches oder mehrbasiges organisches Salz von Magnesium, die sich von Nitrit oder Perjodat unterscheiden, und in dem zur Färbung geeigneten Milieu löslich sind, sowie mindestens ein Derivat von 5,6-Dihydroxyindol der Formel (I): 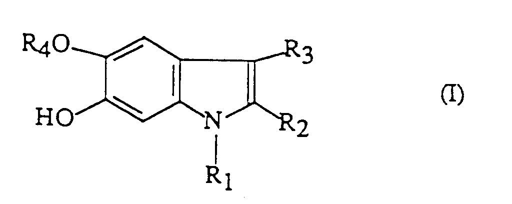 worin R₁, R₂, R₃ und R₄ gleich oder verschieden, Wasserstoff oder einen C₁₋₄-Niedrigalkylrest bedeuten, wobei R₁, R₂ und R₃ Wasserstoff bedeuten, wenn R₄ einen C₁₋₄-Niedrgalkylrest bedeutet, in einem zur Färbung keratinischer Fasern und insbesondere der menschlichen Haare geeigneten Milieu enthält.

17. Zusammensetzung gemäß Anspruch 16,
dadurch **gekennzeichnet**, daß
das Magnesiumsalz das Salz einer Säure ist, die aus halogenierten Wasserstoffsäuren, aus von Schwefel abgeleiteten Oxisäuren, aus Mono-, Di- oder Tricarboxylsäuren, die mindestens 8 Kohlenstoffatome enthalten und gegebenenfalls hydroxyliert sind, und aus organischen Sulfonsäuren mit mindestens 8 Kohlenstoffatomen ausgewählt ist.
